(19) Europäisches Patentamt / European Patent Office / Office européen des brevets

(11) **EP 2 415 485 A1**

(12) **EUROPEAN PATENT APPLICATION**
published in accordance with Art. 153(4) EPC

(43) Date of publication:
**08.02.2012 Bulletin 2012/06**

(51) Int Cl.:
*A61K 47/32* (2006.01)  *A61K 9/48* (2006.01)
*A61K 47/02* (2006.01)  *A61K 47/10* (2006.01)
*A61K 47/12* (2006.01)  *A61K 47/34* (2006.01)
*A61K 47/36* (2006.01)  *A61K 47/38* (2006.01)

(21) Application number: **10758901.2**

(22) Date of filing: **02.04.2010**

(86) International application number:
**PCT/JP2010/056102**

(87) International publication number:
**WO 2010/114134 (07.10.2010 Gazette 2010/40)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **03.04.2009  JP 2009091000**

(71) Applicant: **Nisshin Kasei Co., Ltd.
Osaka 541-0045 (JP)**

(72) Inventors:
• **MORIUCHI, Toshiaki
  Yamatokoriyama-shi
  Nara 639-1058 (JP)**
• **TAGUCHI, Makoto
  Kadoma-shi
  Osaka 571-0078 (JP)**
• **KOJO, Akane
  Kyoto-shi
  Kyoto 603-8212 (JP)**
• **YOSHINO, Hiroyuki
  Kobe-shi
  Hyogo 651-1513 (JP)**
• **FUKUMORI, Yoshinobu
  Kakogawa-shi
  Hyogo 675-0131 (JP)**
• **HAYASHI, Yusuke
  Kobe-shi
  Hyogo 651-1301 (JP)**

(74) Representative: **Perrey, Ralf
  Müller-Boré & Partner
  Grafinger Strasse 2
  81671 München (DE)**

(54) **HARD CAPSULE**

(57)    The present invention provides a hard capsule that exhibits excellent stability even when filled with a solvent for dissolving a poorly soluble drug, and that achieves improved moldability and drying rate. As such a hard capsule, the present invention provides a hard capsule having a film containing (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one water-soluble polymer selected from the group consisting of cellulose polymers, dextrin, and polyvinylpyrrolidone.

EP 2 415 485 A1

**Description**

Technical Field

The present invention relates to a hard capsule comprising a film that comprises a polymer or copolymer obtained by polymerizing or copolymerizing at least one polymerizable vinyl monomer in the presence of polyvinyl alcohol and/or a derivative thereof and a specific compound.

Background Art

A large number of the active substances of medicines, i.e., pharmaceutical active ingredients, are poor in water solubility. Such substances are poorly absorbed from the alimentary tract, and the bioavailability and drug efficacy expression are thus easily reduced or are subject to fluctuation. In preclinical tests, to evaluate drug efficacy or obtain biopharmaceutical parameters using animals or the like, it is common to dissolve a pharmaceutical active ingredient in some solvent to make it more easily absorbed. For a poorly soluble pharmaceutical active ingredient, a polyethylene glycol having relatively low molecular weight and a derivative thereof, a polyoxyethylene sorbitan fatty acid ester, a fatty acid having 6 to 12 carbon atoms or a salt thereof, polyoxyethylene castor oil, a diethylene glycol derivative, or the like may be used. However, these solvents are usually in liquid form, and forming them into tablets is difficult. Therefore, additional consideration must be given to the ultimate dosage form for sale in the market. If these solvents could be directly formulated into pharmaceutical preparations, the time required for the formulation could be greatly shortened. A capsule is highly anticipated to serve as such a dosage form.

Capsules hitherto known are those produced using gelatin or a cellulose derivative as a base material. When a known gelatin hard capsule is filled with a polyethylene glycol having a weight average molecular weight of 400 (PEG 400), the moisture in the capsule film migrates into the solvent, causing the capsule to break (see Non-Patent Literature (NPL) 1). In known cellulose derivative-based capsules, the aforementioned solvents act as plasticizers, causing them to permeate the capsule film and be exuded to the capsule surface.

In order to solve such problems, a hard capsule that is made mainly of a polymer or copolymer obtained by polymerizing or copolymerizing at least one specific polymerizable vinyl monomer in the presence of polyvinyl alcohol and/or a derivative thereof (hereinafter, sometimes referred to as a "PVA copolymer") has been reported (see Patent Literature (PTL) 1).

However, a starting solution for preparing a capsule that is made mainly of a PVA copolymer has high viscosity, and the drying rate is low. Therefore, the production process thereof was not necessarily efficient.

A hard capsule is generally prepared by immersing a molding pin having a hemispherical end in a starting solution for preparing a capsule; slowly withdrawing the immersed pin therefrom to form, at the surface of the pin, a film of the starting solution with a uniform thickness; and drying and solidifying the formed film.

For example, when a gelatin capsule is formed, a starting solution is maintained at a gelation temperature or more, so as to ensure the flowability of the solution during immersion and withdrawal of a molding pin. After the molding pin is withdrawn from the solution, the solution adhering to the pin is cooled to gel, and dried at a gelation temperature or lower. Thereby, a capsule having a homogeneous film thickness can be obtained.

However, a PVA copolymer solution that is used as a starting solution for preparing a capsule that is made mainly of a PVA copolymer has no gelation ability. Further, as mentioned above, the solution suffers from relatively high viscosity and low drying rate. Due to such drawbacks, immersion and withdrawal of a molding pin is difficult; dripping occurs while a capsule film is being formed or being dried; and drying takes a long time. To overcome these and other problems, there has been a demand for further improvement in capsule moldability and efficiency of the production of the capsule.

Citation List

Patent Literature

    PTL 1: WO 2002/017848

Non-Patent Literature

NPL 1: Pharmaceutical Technology Europe, October, 84, 86, 88-90, 1998

Summary of Invention

Technical Problem

The principal object of the present invention is to provide a capsule that has a feature of a capsule made mainly of a PVA copolymer, i.e., excellent stability when filled with a poorly soluble drug-dissolving solvent; and that also achieves improved moldability and drying performance in the preparation of the capsule.

Solution to Problem

The present inventors conducted extensive research to achieve the above object, and found the following. A capsule comprising a film that comprises a specific water-soluble polymer and a polymer or copolymer (a PVA copolymer) obtained by polymerizing or copolymerizing at least one specific polymerizable vinyl monomer in the presence of polyvinyl alcohol and/or or a derivative thereof exhibits high stability even when filled with a poorly soluble pharmaceutical active ingredient-dissolving solvent; and has general characteristics that hard capsules are required to have, such as water solubility. The present inventors further found that the above-mentioned capsule also achieves improvement in moldability and drying performance. The inventors conducted further research, and accomplished the present invention.
More specifically, the present invention encompasses the following hard capsule and a method for producing the capsule.

Item 1. A hard capsule having a film comprising:

(A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof; and

(B) at least one water-soluble polymer selected from the group consisting of cellulose polymers, dextrin, and polyvinylpyrrolidone.

Item 2. The hard capsule according to Item 1, wherein the water-soluble polymer of (B) is at least one member selected from the group consisting of hydroxypropyl methyl cellulose, hydroxypropyl cellulose, methyl cellulose, hydroxyethyl cellulose, dextrin, and polyvinylpyrrolidone.

Item 3-1. The hard capsule according to Item 1 or 2, comprising 2 to 100 parts by weight of the water-soluble polymer of (B), relative to 100 parts by weight of the polymer or copolymer of (A).

Item 3-2. The hard capsule according to any of Items 1 to 3-1, wherein the film comprises 40 to 99 wt% of the polymer or copolymer of (A), on a dry weight basis, relative to the total weight of the film.

Item 3-3. The hard capsule according to any of Items 1 to 3-2, wherein the film comprises 1 to 60 wt% of the water-soluble polymer of (B), on a dry weight basis, relative to the total weight of the film.

Item 4. The hard capsule according to any of Items 1 to 3-3, wherein the film further comprises (C) a gelling agent.

Item 5. The hard capsule according to any of Items 1 to 4, which is to be filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,

(f) aliphatic alcohols having 6 to 12 carbon atoms, and
(g) polyoxyethylene sorbitol fatty acid esters.

Item 6. A hard capsule formulation comprising the hard capsule of any of Items 1 to 5, wherein the hard capsule is filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,
(f) aliphatic alcohols having 6 to 12 carbon atoms, and
(g) polyoxyethylene sorbitol fatty acid esters.

Item 7. A method for producing a film of the hard capsule of any of Items 1 to 5, comprising:

forming an aqueous solution containing

(A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and
(B) at least one water-soluble polymer selected from the group consisting of cellulose polymers, dextrin, and polyvinylpyrrolidone,

into a capsule form by drying.

Item 8. Use of (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof; and (B) at least one water-soluble polymer selected from the group consisting of cellulose polymers, dextrin, and polyvinylpyrrolidone, in producing a hard capsule.

Advantageous Effects of Invention

The hard capsule of the present invention has a feature in that it has excellent stability even when filled with a poorly soluble drug-dissolving solvent. In addition, the hard capsule of the present invention is advantageous in that it achieves improved operability, moldability, and drying performance in the preparation of the capsule. Therefore, efficiency of the production of the capsule also improves. Further, weakening in strength of the capsule under high humidity is inhibited, and the dissolution rate of the capsule is also improved.
Specifically, the present invention provides a hard capsule that can be filled with various types of pharmaceutical active ingredients that have been considered unsuitable for filling a capsule. The hard capsule provided by the present invention can also achieve excellent operability, moldability, drying performance, and efficiency of the production. Therefore, the hard capsule of the present invention contributes to the practical utilization of various types of drugs, and improvement in quality of capsule formulation.

Brief Description of Drawings

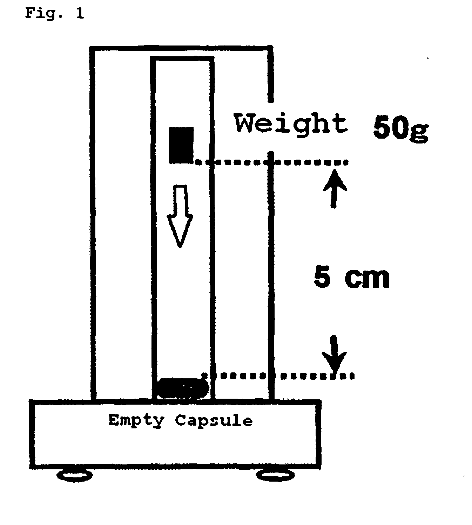

Figure 1 schematically illustrates an impact strength testing machine for hard capsules.

Description of Embodiments

Hereinafter, the present invention is described in more detail.

1. Film

It is essential that the film of the hard capsule of the present invention comprises (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one water-soluble polymer selected from the group consisting of cellulose polymers, dextrin, and polyvinylpyrrolidone.

(A) A polymer or copolymer obtained by polymerizing or copolymerizing at least one polymerizable vinyl monomer represented by Formula (1): $H_2C = C(R_1)\text{-}COOR_2$ (1), wherein $R_1$ represents hydrogen or methyl, and $R_2$ represents hydrogen or alkyl having 1 to 4 carbon atoms, in the presence of polyvinyl alcohol and/or a derivative thereof Polyvinyl alcohols (sometimes referred to as PVA) and derivatives thereof usable in the present invention are completely saponified PVA, intermediately saponified PVA, partially saponified PVA, as well as various modified PVAs, such as amine-modified PVA, ethylene-modified PVA, terminal-thiol-modified PVA, and the like.

PVAs can be obtained by radical-polymerizing vinyl acetate, and suitably saponifying the obtained vinyl acetate. Therefore, PVAs generally have -OCOCH$_3$ groups originating from vinyl acetate. PVAs can be classified into those that are completely saponified, intermediately saponified, partially saponified, and the like, depending on the degree of saponification. PVAs that are usable in the present invention preferably have a saponification degree of about 70 mol% or more, more preferably about 80 mol% or more, and still more preferably 85 mol% or more. Of these, saponificated PVAs with a saponification degree of 85 to 90 mol%, and in particular about 86 to 89 mol%, are preferable. As is well known in this field, completely saponified PVA generally refers to PVA with a saponification degree of 98 mol% or more, and does not necessarily indicate PVA with a saponification degree of 100 mol%.

Examples of PVA derivatives include various kinds of modified PVAs, such as amine-modified PVA, ethylene-modified PVA, and terminal-thiol-modified PVA. These modified PVAs may be produced by, for example, methods known in this field.

Commercially available PVAs and derivatives thereof may also be used. They may be purchased from, for example, Nippon Synthetic Chemical Industry Co., Ltd., Japan Vam &. Poval Co., Ltd., or the like.

PVAs are known to have various average molecular weights and polymerization degrees. The optimum viscosity of PVAs varies depending on the method for producing a hard capsule, and PVAs usable therefor can also be suitably selected.

As one embodiment, PVAs usable in the present invention are those having a weight average molecular weight of about 30,000 to 400,000, and preferably about 100,000 to 300,000. The weight average molecular weight of PVA is a value measured by a GPC method (nonaqueous size exclusion chromatography). Specifically, the weight average molecular weight is measured as follows: PVA is dissolved in dimethyl sulfoxide (DMSO) containing lithium chloride at a concentration of 10 moL, so that the concentration of the PVA is 1 mg/mL; the mixture is stirred while heating at 40°C for 30 minutes, and then left to stand at room temperature overnight; the resulting product is filtrated through a PTFE cartridge filter (0.45 μm), followed by measurement of the molecular weight distribution by a GPC method.

In addition, PVAs having an average polymerization degree of, for example, about 350 to 5,000, and preferably about 1,200 to 3,800, can be used in the present invention.

When PVAs having the above-mentioned weight average molecular weights and average polymerization degrees are used, it is preferable to employ, in particular, a dipping method to produce a hard capsule.

Polymerizable vinyl monomers usable in the present invention are specific compounds represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms.

Specific examples of the polymerizable vinyl monomers usable in the present invention include acrylic acid, methacrylic acid, methyl methacrylate, methyl acrylate, ethyl methacrylate, ethyl acrylate, butyl methacrylate, butyl acrylate, isobutyl methacrylate, and isobutyl acrylate. Salts of acrylic acid or methacrylic acid can also be used. Examples of such salts include sodium salt, potassium salt, ammonium salt, and alkylamine salt.

The polymerizable vinyl monomers may be used singly or in a combination of two or more.

As the polymerizable vinyl monomers, it is preferable to use at least one of acrylic acid and methacrylic acid, in combination with at least one member selected from the group consisting of methyl methacrylate, methyl acrylate, ethyl methacrylate, ethyl acrylate, butyl methacrylate, butyl acrylate, isobutyl methacrylate, and isobutyl acrylate. It is more preferable to use acrylic acid or methacrylic acid, in combination with methyl methacrylate.

In the polymer or copolymer (i.e., the PVA copolymer of (A)) of the present invention obtained by polymerizing or copolymerizing at least one polymerizable vinyl monomer, in the presence of polyvinyl alcohol and/or a derivative thereof, the ratio of the PVA and/or derivative thereof to the polymerizable vinyl monomer is not particularly limited. However, it is preferable that the PVA and/or derivative thereof be used in an amount of 20 to 95 wt%, and the polymerizable vinyl monomer be used in an amount of 5 to 80 wt%. It is more preferable that the PVA and/or derivative thereof be used in an amount of 50 to 90 wt%, and the polymerizable vinyl monomer be used in an amount of 10 to 50 wt%.

It is preferable that the PVA and/or derivative thereof be used in an amount of 20 wt% or greater, rather than less than 20 wt%, because when the amount of the PVA and/or derivative thereof is 20 wt% or greater, the produced capsule shows more improved dissolution or dispersion ability in water. In addition, when the amount of the PVA and/or derivative thereof is 95 wt% or less, the produced capsule is less easily affected by humidity so that the capsule is not easily weakened in strength under high humidity, compared with the case where the amount of the PVA and/or derivative thereof exceeds 95 wt%.

When two or more polymerizable vinyl monomers are used in combination, the ratio is not particularly limited. However, when at least one member selected from the group (I) consisting of acrylic acid and methacrylic acid; and sodium salts, potassium salts, ammonium salts, and alkylamine salts, of acrylic acid and methacrylic acid is used in combination with at least one member selected from the group (II) consisting of methyl methacrylate, methyl acrylate, ethyl methacrylate, ethyl acrylate, butyl methacrylate, butyl acrylate, isobutyl methacrylate, and isobutyl acrylate, the weight ratio thereof is as follows: the group (I) is used in an amount of 5 to 50 wt%, and preferably 10 to 40 wt%, and the group (II) is used in an amount of 50 to 95 wt%, and preferably 60 to 90 wt%, relative to the total amount of the polymerizable vinyl monomers.

A known method may be used for the copolymerization. For example, a PVA and/or derivative thereof is added to water, and the mixture is heated to effect dissolution. Then, at least one polymerizable vinyl monomer and a polymerization initiator are added thereto to initiate the copolymerization, thereby obtaining a resin. For example, a PVA and/or derivative thereof is dispersed in ion exchange water, and allowed to completely dissolve at 90 to 100°C. Then, at least one polymerizable vinyl monomer is added thereto, and after purging with nitrogen, a polymerization initiator is added to conduct a reaction for about 2 to 5 hours. The weight ratio of the PVA and/or derivative thereof to the polymerizable vinyl monomer, in the PVA copolymer of (A), is determined according to the weight ratio of those added to water, i.e., the PVA and/or derivative thereof to the polymerizable vinyl monomer. Therefore, when added to water, the weight ratio of the PVA and/or derivative thereof to polymerizable vinyl monomer is preferably equal to the above-mentioned weight ratio in the PVA copolymer of (A).

Usable polymerization initiators are those hitherto used. Examples thereof include 2,2'-azobis(2-amidinopropane) hydrochloride, AIBN (azoisobutyronitrile), and like azo compounds; potassium persulfate, sodium persulfate, ammonium persulfate, and like persulfates; t-butyl hydroperoxide and like organic peroxides; and hydrogen peroxide-tartaric acid, hydrogen peroxide-sodium tartrate, and like redox initiators.

The PVA copolymer of (A) preferably has an average polymerization degree of about 350 to 5,000, and more preferably about 1200 to 3800. The PVA copolymer of (A) preferably has a weight average molecular weight of about 30,000 to 400,000, and more preferably about 100,000 to 300,000. This weight average molecular weight is measured in the same manner as in the above-mentioned method for measuring the weight average molecular weight of a PVA (a GPC method).

The amount of the PVA copolymer of (A) is, on a dry weight basis, preferably about 40 to 99 wt%, more preferably about 50 to 98 wt%, and still more preferably about 60 to 95 wt%, relative to the total weight of the film.

According to the present invention, although restrictive interpretation is not intended, the reaction mechanism of the polymerization or copolymerization of at least one specific polymerizable vinyl monomer in the presence of polyvinyl alcohol and/or a derivative thereof is assumed to be as follows: first, a polymerization initiator abstracts hydrogen from the methyl group at the terminal of -OCOCH$_3$ present in the PVA, creating a radical. Then, the polymerizable vinyl monomer bonds to the radical, allowing the double bond of the polymerizable vinyl monomer to be cleaved, thereby again creating a radical. Then, the polymerizable vinyl monomer bonds to the radical; the reaction is repeated in the same manner as above.

In the present invention, the PVA copolymer of (A) has a structure in which at least one of the aforementioned polymerizable vinyl monomers is graft polymerized with -OCOCH$_3$, which is a side chain of PVA. In this graft polymerization, the PVA may be joined together through a polymer obtained by polymerization or copolymerization of at least one of the polymerizable vinyl monomers.

For example, when acrylic acid and methyl methacrylate are used as polymerizable vinyl monomers, the PVA copolymer of (A) has a structure in which a copolymer of acrylic acid and methyl methacrylate is bonded to PVA through

-OCOCH$_3$ of the PVA. Specific examples of such PVA copolymers (copolymers of polyvinyl alcohol/acrylic acid/methyl methacrylate) include POVACOAT® Type R and POVACOAT® Type L (produced by Daido Chemical Corporation), which are used in the Examples described below.

(B) Water-soluble polymer

The film of the hard capsule of the present invention further comprises at least one water-soluble polymer selected from the group consisting of cellulose-based polymers (i.e., cellulose derivatives), dextrin, and polyvinylpyrrolidone (PVP).

Examples of cellulose-based polymers include hydroxypropyl methylcellulose (also called hypromellose in the Japanese Pharmacopoeia; hereinafter also referred to as "HPMC"), hydroxypropyl cellulose (hereinafter also referred to as "HPC"), methylcellulose (hereinafter also referred to as "MC"), and hydroxyethylcellulose (hereinafter also referred to as "HEC").

These may be used singly, or in a combination of two or more.

The viscosity of the cellulose-based polymers is not particularly limited, and is preferably about 3 to 7 mPa·s in a 2% aqueous solution at 20°C. When, in particular, HPMC is used as a cellulose-based polymer, there is no limitation on the amounts of substituents introduced; and a methoxy content is preferably 28 to 30 mass%, and a hydroxy propoxy content is preferably 7 to 12 mass%.

Among water-soluble polymers, cellulose-based polymers are particularly preferable, because when a cellulose-based polymer is used to produce a hard capsule that is made mainly of the PVA copolymer, the thus-produced capsule maintains excellent stability when filled with a poorly soluble drug-dissolving solvent; and the moldability of the capsule and the efficiency of the production of the capsule are also excellent.

HPMC is particularly preferable, because it prevents a hard capsule that is made mainly of a PVA copolymer from weakening in strength under high humidity.

Such water-soluble polymers are known, or otherwise can be easily produced by a known method. Commercially available products, which may be purchased from, for example, Shin-Etsu Chemical Co., Ltd., and Nippon Soda Co., Ltd., can also be used.

The amount of the water-soluble polymer of (B) is preferably, on a dry weight basis, about 1 to 60 wt%, more preferably about 2 to 50 wt%, even more preferably about 5 to 40 wt%, relative to the total weight of the film.

When the amount of the water-soluble polymer is 1 wt% or more, the drying rate of the capsule is significantly improved, and dripping of the starting solution for producing a capsule is efficiently prevented. When the amount of the water-soluble polymer is 60 wt% or less, the resulting capsule exhibits stability even when filled with a poorly soluble pharmaceutically effective ingredient-dissolving solvent, and leakage of the filled material from the hard capsule is less likely to occur.

The film of the hard capsule contains the water-soluble polymer of (B) in an amount of preferably 2 to 100 parts by weight, and more preferably 5 to 70 parts by weight, relative to 100 parts by weight of the PVA copolymer of (A).

When the water-soluble polymer of (B) is used to form a capsule film, the viscosity of the starting solution can be reduced, and appropriate flowability can be imparted to the starting solution. This allows easy immersion and withdrawal of a molding pin, improving the operability in the formation of a capsule. Further, dripping of the starting solution is prevented at the time of forming and drying a capsule, thereby improving the moldability of the capsule. Additionally, the drying rate for drying a capsule is accelerated, improving the drying property.

As a result of improvements in the operability, moldability, and drying property, the speed for producing a capsule increases, improving the efficiency of the production of a capsule.

(C) Other components

The film may also contain one or more other components in addition to (A) and (B) above, as long as such components do not impair the effects of the present invention.

For example, the film may contain a known plasticizer. Examples of plasticizers include polyhydric alcohols. Specific examples of polyhydric alcohols include glycerol, ethylene glycol, diethylene glycol, triethylene glycol, polyethylene glycol, propylene glycol, polypropylene glycol, diglycerol, 1,3-butylene glycol, and sugar alcohols. Examples of sugar alcohols include sorbitol and mannitol. Of these, glycerol, propylene glycol, sorbitol, and mannitol are preferable, and glycerol and propylene glycol are more preferable. These may be used singly, or in a combination of two or more.

The film may also contain an ester of polyhydric alcohol. As the esters of polyhydric alcohols, for example, an ester of a polyhydric alcohol and a carboxylic acid having 1 to 5 carbon atoms, preferably 1 to 4 carbon atoms, and more preferably 1 to 3 carbon atoms, is used. Preferable examples thereof include monoesters, diesters, triesters, etc., of the aforementioned polyhydric alcohols. Specific preferable examples of the esters of polyhydric alcohols include glycerol triacetate (hereinafter sometimes referred to as "triacetin"), glycerol monoacetate, glycerol diacetate, glycerol tributyrate, glycerol tripropionate, propylene glycol diacetate, and ethylene glycol dibutyrate. Of these, triacetin is particularly preferable. These may be used singly, or in a combination of two or more.

The film may also contain an ester of polyvalent carboxylic acid. As the esters of polyvalent carboxylic acids, for example, an ester of a polyvalent carboxylic acid and alcohol having 1 to 5 carbon atoms, preferably 1 to 4 carbon atoms, and more preferably 1 to 3 carbon atoms, can be used. For example, monoesters, diesters, triesters, etc., of polyvalent carboxylic acids can be used. The usable polyvalent carboxylic acids are not limited as long as they have two or more carboxyl groups. Specific preferable examples of polyvalent carboxylic acids include citric acid, acetylcitric acid, tartaric acid, malic acid, fumaric acid, maleic acid, malonic acid, glutaric acid, adipic acid, and succinic acid. Specific preferable examples of the esters of polyvalent carboxylic acids include triethyl citrate, tributyl citrate, acetyl triethyl citrate, diethyl succinate, and dimethyl succinic acid. Of these, triethyl citrate is particularly preferable. These may be used singly, or in a combination of two or more.

Further, a water-soluble polymer other than the above may also be included in the film to an extent that does not impair the effects of the present invention. Examples of such water-soluble polymers include natural polysaccharides, semisynthetic polysaccharides, proteins, and synthetic polymers.

Examples of natural polysaccharides include agar, mannan, pullulan, starches (e.g., corn starch, potato starch, wheat starch, and rice starch), pregelatinized starch, amylose, and dextran.

Examples of semisynthetic polysaccharides include hydroxypropyl starch, hydroxyethyl starch, and cyclodextrin polymers.

Examples of proteins include gelatin, casein, and zein.

Examples of synthetic polymers include polyoxyethylene polyoxypropylene glycols, carboxyvinyl polymers, and polyethylene glycols.

These may be used singly, or in a combination of two or more.

It is also possible to add a known so-called gelling agent. Specific examples thereof include kappa carrageenan, iota carrageenan, lambda carrageenan, tamarind seed polysaccharide, pectin, curdlan, gelatin, furcellaran, agar, xanthan gum, locust bean gum, and gellant gum. These may be used singly, or in a combination of two or more.

A gelling aid may also be added, if necessary. Examples of gelling aids include water-soluble compounds containing potassium ions, ammonium ions, or calcium ions. Examples of such water-soluble compounds include potassium chloride, potassium phosphate, calcium chloride, and ammonium chloride. A gelling aid may be suitably selected according to the type of the gelling agent to be used. With kappa carrageenan, for example, potassium chloride, potassium phosphate, calcium chloride, ammonium chloride, or the like, can be used as the gelling aid.

It is also possible to add a dye, a pigment, and like colorants; an opacifying agent; a flavor; sodium lauryl sulfate and like surfactants; and the like, within a range that does not hinder the effects of the present invention.

The amount of one or more of the other components (C) is suitably adjusted within a range that enables the production of the hard capsule. For example, the amount of a gelling agent is not limited insofar as the effects of the present invention are not impaired; and is generally, on a dry weight basis, about 0.05 to 10.0 wt%, preferably about 0.10 to 3.00 w%, and more preferably about 0.2 to 1.0 wt%, relative to the total weight of the film. The amount of a gelling aid is also not limited insofar as the effects of the present invention are not impaired, and is generally about 0.05 to 10.0 wt%, preferably about 0.10 to 3.00 wt%, and more preferably about 0.2 to 1.0 wt%, relative to the total weight of the film. Further, the amount of the above-mentioned plasticizer, ester of polyhydric alcohol, and ester of polyvalent carboxylic acid is generally, on a dry weight basis, about 1 to 20 wt%, preferably about 2 to 15 wt%, and more preferably about 3 to 10 wt%, relative to the total weight of the film. Note that when at least two members selected from the group consisting of the above-mentioned plasticizers, esters of polyhydric alcohols, and esters of polyvalent carboxylic acids are used in combination, it is preferable that the amount of the combination falls within the above-mentioned amount range.

The thickness of the film of the hard capsule is not particularly limited, as long as the functions as a hard capsule are satisfactory, and is generally about 0.01 to 5 mm, preferably about 0.05 to 1 mm, and more preferably about 0.05 to 0.5 mm.

## 2. Production method

The hard capsule comprising a film of the present invention may be produced by, for example, an injection molding method, or a dipping method. The production method is not particularly limited to the above as long as a hard capsule can be formed. The methods that are used to produce general hard gelatin capsules may also be used. It is preferable to employ a dipping method.

A dipping method produces a capsule by using the fact that a hard capsule base material turns into a gel due to a temperature difference. When the base material does not exhibit gelation ability, an aforementioned gelling agent, and further, an aforementioned gelling aid, if necessary, can also be added to produce a hard capsule.

An embodiment is given below with respect to a method for producing a hard capsule by using a gelling agent. Starting materials, which are (A) a polymer or copolymer obtained by polymerizing or copolymerizing at least one polymerizable vinyl monomer in the presence of polyvinyl alcohol and/or a derivative thereof, (B) a water-soluble polymer, (C) a gelling agent, and optionally, (D) a gelling aid, are dissolved in water, thereby preparing a starting solution for producing a capsule. A molding pin is immersed in the prepared starting solution and withdrawn therefrom, followed by gelling and drying the solution adhering to the pin to form a film. The order for dissolving the starting materials is not particularly limited, as long as a starting solution for preparing the capsule of the present invention can be obtained. For example, after (B) is dissolved in water, (A), (C), and (D) can be dissolved in water in this order. When these materials are dissolved, the water can be suitably stirred. It is preferable to appropriately heat the water. When heating is performed, the heating temperature is preferably about 50 to 90°C. More specifically, the methods described in the Examples are exemplified.

The concentration of each of the starting materials in the starting solution for preparing a capsule is not limited as long as the capsule of the present invention can be obtained; and is suitably adjusted. For example, it is preferable that the concentration of (A) and (B) (total concentration) be 10 to 30 wt%.

The starting solution for preparing a capsule contains (B) in an amount of preferably 2 to 100 parts by weight, and more preferably 5 to 70 parts by weight, relative to 100 parts by weight of (A).

A solution containing a water-soluble polymer (in particular, HPMC, HPC, etc.) of (B) has a property such that the water-soluble polymer dissolves at a lower temperature and the viscosity of the solution thereby increases. Therefore, in producing a capsule by a dipping method, the starting solution for preparing a capsule that is heated (to, for example, 50 to 90°C) has a low viscosity; and when a molding pin is withdrawn from the starting solution, the temperature of the solution adhering to the pin decreases while the viscosity thereof increases. For this reason, dripping of the solution can be prevented when the molding pin is withdrawn from the solution (i.e., the moldability of a capsule improves); the drying rate significantly improves (i.e., the drying property improves); and in addition, due to the low viscosity of the starting solution, appropriate flowability can be obtained, allowing easy immersion and withdrawal of a molding pin (i.e., the operability improves). Because the starting solution has a low viscosity, the solids concentration of the starting solution can also be increased without impairing the operability. As a result, even when the starting solution that is made to adhere to a molding pin is used in a small amount, a thick capsule can be produced, allowing the production of a capsule to be performed more easily.

### 3. Hard capsule formulation

The present invention also encompasses a hard capsule formulation in which a hard capsule comprising the above-described film is filled with content.

There is no particular limitation on the form of contents used to fill the capsule; and the contents may be, for example, in the form of a liquid, a powder, granules, a paste, a semi-solid or ointment, or a cream. The capsule of the present invention is preferably used for enclosing, in particular, a poorly soluble drug-dissolving solvent.

As described above, a poorly soluble drug-dissolving solvent refers to a solvent that dissolves a poorly soluble drug. Poorly soluble drugs refer to those having poor water solubility, and may be any of those defined as "Sparingly soluble", "Slightly soluble", "Very slightly soluble", or "Practically insoluble or insoluble", as described in the Japanese Pharmacopoeia Fifteenth Edition. Specifically, the degree of dissolution within 30 minutes is evaluated by forming a drug into a powder when the drug is a solid, and then vigorously shaking the powder in water at 20±5°C for 30 seconds at 5-minute intervals. When the amount of water required to dissolve 1 g or 1 mL of a drug is 30 mL or more and less than 100 ml, the drug is evaluated as "Sparingly soluble"; when the amount is 100 mL or more and less than 1,000 mL, the drug is evaluated as "Slightly soluble"; when the amount is 1,000 mL or more and less than 10,000 mL, the drug is evaluated as "Very slightly soluble"; and when the amount is 10,000 mL or more, the drug is evaluated as "Practically insoluble or insoluble".

There is no particular limitation on poorly soluble drug-dissolving solvents, as long as they are pharmaceutically acceptable, and can dissolve poorly soluble drugs. Examples of poorly soluble drug-dissolving solvents include polyethylene glycols and derivatives thereof, diethylene glycol ether derivatives, propylene glycol fatty acid esters, glycerin fatty acid esters, polyglyceryl fatty acid esters, polyoxyethylene glycerin fatty acid esters, sorbitan fatty acid esters, polyoxyethylene sorbitan fatty acid esters, polyoxyethylene sorbitol fatty acid esters, polyoxyethylene castor oil, medium chain fatty acids and salts thereof, and medium chain aliphatic alcohols.

As polyethylene glycols, those having a low molecular weight are preferable. Examples thereof include polyethylene glycols having a weight average molecular weight of 2,000 or less, preferably 1,500 or less, and more preferably 1,000 or less. Specific examples thereof include PEG 400 (a polyethylene glycol having a weight average molecular weight of about 400). Examples of the derivatives thereof include fatty acid ester derivatives. The weight average molecular weights of polyethylene glycols are values measured in the following manner. Specifically, 42 g of phthalic anhydride is added to a 1-L ground-in stopper bottle that is protected from light and that contains 300 mL of newly distilled pyridine. The resulting product is vigorously shaken to effect dissolution, and then left to stand for 16 hours or more. Thereafter, 25 mL of the obtained liquid is introduced into a pressure resistant ground-in stopper bottle (about 200 mL), followed by the addition of about 0.8 to 15 g of a PEG sample to be measured. The resulting bottle is sealed, enclosed in durable fabric, and placed in a water bath that has been heated to $98\pm2°C$ in advance. At this time, the bottle is placed in the water bath so that the liquid in the bottle is immersed in the water bath. After the temperature is kept at $98\pm2°C$ for 30 minutes, the bottle is taken out of the water bath, and cooled in air to room temperature. Subsequently, 50 mL of 0.5 mol/L sodium hydroxide liquid is added thereto, followed by further addition of five drops of a pyridine solution of phenolphthalein $(1 \rightarrow 100)$. The resulting liquid is titrated with 0.5 mol/L sodium hydroxide liquid, provided that the titration is terminated when the liquid shows a pale red color continuously for 15 seconds. A blank experiment is carried out in a manner similar to the above. The weight average molecular weight is calculated using the following formula:

$$\text{Average molecular weight} =$$

$$(\text{the amount of sample (g)} \times 4,000/(a-b))$$

a: the amount (mL) of 0.5 mol/L sodium hydroxide liquid consumed in the blank experiment
b: the amount (mL) of 0.5 mol/L sodium hydroxide liquid consumed in the experiment of PEG sample

Examples of medium chain fatty acids and salts thereof include fatty acids having 6 to 12 carbon atoms and salts thereof. Specific examples thereof include caproic acid, caprylic acid, capric acid, and lauric acid, and sodium salts and potassium salts of these acids.

Examples of medium chain aliphatic alcohols include aliphatic alcohols having 6 to 12 carbon atoms. Specific examples thereof include caproyl alcohol, capryl alcohol, and lauryl alcohol.

The poorly soluble drug-dissolving solvents can be used singly, or in a combination of two or more.

Solvents to be filled in the hard capsule of the present invention are not limited to only poorly soluble drug-dissolving solvents, as long as they are pharmaceutically acceptable solvents that can dissolve drugs. It is also possible to use a mixture of a poorly soluble drug-dissolving solvent and one or more other known solvents.

Filling a known hard capsule with a poorly soluble drug-dissolving solvent would cause breakage, etc., of the capsule. Therefore, dissolving a poorly soluble drug with a solvent, and filling a capsule therewith was difficult. However, the hard capsule comprising the film of the present invention is not easily broken even when filled with a poorly soluble drug-dissolving solvent; therefore, dissolving of a poorly soluble drug with a solvent, and filling of the capsule therewith can be performed.

The above-mentioned poorly soluble drug-dissolving solvents may contain a thickener. The addition of a thickener can achieve the following effects: the operation of filling the hard capsule with a solvent is simplified; and leakage of the filled material from the hard capsule is prevented. There are no particular limitations on thickeners, as long as they are those that are pharmaceutically acceptable, such as light anhydrous silicic acid, vegetable oils, and cellulose derivatives (e.g., those described in pharmaceutical textbooks, or those generally used). The amount of thickener added is preferably, for example, 0.1 to 10 parts by weight, more preferably 0.3 to 3 parts by weight, relative to 100 parts by weight of the poorly soluble drug-dissolving solvent.

The above-mentioned poorly soluble drug-dissolving solvent may further contain an additive that can generally be added to a solvent used to fill a capsule, as long as such an additive does not impair the functions of the capsule. Examples of such additives include lactose and starches.

Drugs (including poorly soluble drugs) used to fill the hard capsule of the present invention are not limited due to its application. As medicines, for example, the following can be filled in the hard capsule of the present invention: vitamins, antifebriles, analgesics, antiphlogistics, antiulcer drugs, cardiotonics, anticoagulants, hemostatic agents, bone resorption inhibitors, vascularization inhibitors, antidepressants, antitumor agents, antitussives/expectorants, muscle relaxants, antiepileptics, antiallergic agents, antiarrhythmics, vasodilators, antihypertensive diuretics, diabetes drugs, antituberculous agents, hormonal agents, antinarcotics, antibacterials, antifungals, antivirals, and the like. These may be used in a solid form (for example, in the form of a powder or granules) to fill the capsule, or may be dissolved in a solvent to fill the capsule.

Example of poorly soluble drugs include agrigine, ajmaline, amobarbital, chlordiazepoxide, chlormadinone acetate, clonazepam, diazepam, diltiazem, kitasamycin, dicumarol, sulfathiazole, medazepam, menadione, midecamycin, piroxicam, nystatin, phenacetin, phenobarbital, phenothiazine, flunitrazepam, prednisolone, nicergoline, phenytoin, probucol, nifedipine, reserpine, furosemide, glibenclamide, indomethacin, griseofulvin, nitrazepam, albendazole, carbamazepine, and phenylbutazone.

The above describes dissolving a drug with a poorly soluble drug-dissolving solvent so as to fill the capsule therewith. However, it is surely also possible to fill the capsule with a drug that is not poorly soluble. In this case, such a drug may be used in a solid form (for example, in the form of a powder or granules) to fill the capsule, or may be dissolved in a solvent to fill the capsule.

Commercially available products or those produced by a known method may be used as such drugs.

It is also possible to add other known capsule techniques to the hard capsule formulation of the present invention, as required. For example, if the area where the cap and body of a capsule meet is sealed with, for example, a material similar to the coating film of the capsule, leakage or dissipation of the content can be prevented. The sealing can also be performed using polyvinylpyrrolidone. Specific examples of sealing methods include a band-sealing method.

The hard capsule of the present invention can be used as an inhalation preparation or a pharmaceutical preparation for rectal administration, in addition to use as a pharmaceutical preparation for oral administration. Further, in addition to drugs for medical treatment, the hard capsule of the present invention can also be used in the fields of, for example, food and cosmetics. Specifically, cosmetics or food may be used to fill the capsule of the present invention.

Examples

Hereinafter, the present invention is described in more detail with reference to Examples and Comparative Examples. However, the present invention is not limited to the following Examples.

In the Examples and Comparative Examples, "%" represents "wt%," unless otherwise specified.

The term "Addition Concentration" in the tables represents the weight of the compound of (B) of the present invention, on a dry weight basis, relative to the total weight of the film. Specifically, the term represents the proportion of the weight of (B), when the total weight of the starting materials ((A) to (D)) of the capsule is 100 wt%.

Further, the term "Solids Concentration" refers to the total weight of starting materials ((A) to (D)) of the capsule, relative to the total weight of the starting solution for preparing a capsule.

A "size No. 3" capsule represents a capsule having a capacity of about 0.3 mL, a mass of about 50 mg, a cap length of about 8.23 mm , a body length of about 13.16 mm, and a capsule binded length prior to filling of about 17.7 mm.

1. Production of hard capsule

Production of PVA copolymer

38.1 g of PVA (Type: EG-05; average polymerization degree: 500; saponification degree: 88%; produced by Nippon Synthetic Chemical Industry Co., Ltd.), 89.0 g of PVA (Type: EG-25; average polymerization degree: 1,700; saponification degree: 88%; produced by Nippon Synthetic Chemical Industry Co., Ltd.), and 641 g of ion exchange water, were introduced into a separable flask equipped with a cooling reflux tube, a dropping funnel, a thermometer, a nitrogen inlet tube, and a stirrer. The mixture was dispersed at an ordinary temperature, and then completely dissolved at 95°C. Subsequently, 4.0 g of acrylic acid and 27.8 g of methyl methacrylate were added thereto. After the flask was purged with nitrogen, the temperature was increased to 50°C. Thereafter, 8.5 g of tertiary butyl hydroperoxide and 8.5 g of sodium erythorbate were added thereto, and the reaction was terminated after 4 hours, thereby obtaining a PVA copolymer solution. The obtained PVA copolymer solution was dried and pulverized to yield a PVA copolymer powder. The thus-obtained PVA copolymer powder was used as a PVA copolymer in the following production of capsules.

Production of capsule

(1) 1.7 g of HPMC (TC-5 (registered trademark) R; produced by Shin-Etsu Chemical Co., Ltd.; viscosity: about 6 mPa·s in 2% aqueous solution at 20°C) was dissolved in 166 g of purified water. Then, 32.3 g of the PVA copolymer, 0.34 g of kappa carrageenan, and 0.34 g of potassium chloride were added thereto to yield a starting solution for preparing a capsule. The yielded solution was kept warm at about 60°C, and a stainless steel pin at room temperature was immersed and withdrawn to thereby produce a size No. 3 hard capsule having a film thickness of about 0.06 to 0.15 mm. The hard capsule produced in this manner was named "Example 1."

TC-5R is HPMC (substitution type: 2910), i.e., HPMC containing about 29 mass% of methoxy group and about 10 mass% of hydroxy propoxy group.

(2) A hard capsule of the same size as that obtained in (1) above was produced in the same manner as in (1) above, except that the amount of the HPMC was changed from 1.7 g to 3.4 g, and the amount of the PVA copolymer was changed from 32.3 g to 30.6 g. The hard capsule produced in this manner was named "Example 2."

(3) Hereunder, capsules of Examples 3 and 5 to 12 were produced in the same manner as described above, except that the types and amounts of the additives, and the amount of the PVA copolymer were changed. A capsule of Example 4 was also produced in the same manner as above, except that the amounts of kappa carrageenan, potassium chloride, and purified water were also changed.

Specifically, a hard capsule of the same size as that obtained in (1) above was produced in the same manner as in (1) above, except that the amount of the HPMC was changed from 1.7 g to 5.1 g, and the amount of the PVA copolymer was changed from 32.3 g to 28.9 g. The hard capsule produced in this manner was named "Example 3."

Further, a hard capsule of the same size as that obtained in (1) above was produced in the same manner as in (1) above, except that the amount of the HPMC was changed from 1.7 g to 2.0 g, the amount of the PVA copolymer was changed from 32.3 g to 38.0 g, the amount of purified water was changed from 166 g to 160 g, the amount of kappa carrageenan was changed from 0.34 g to 0.25 g, and the amount of potassium chloride was changed from 0.34 to 0.25 g. The hard capsule produced in this manner was named "Example 4."

A hard capsule of the same size as that obtained in (1) above was produced in the same manner as in (1) above, except that the amount of the HPMC was changed from 1.7 g to 13.6, and the amount of the PVA copolymer was changed from 32.3 g to 20.4 g. The hard capsule produced in this manner was named "Example 5."

A hard capsule of the same size as that obtained in (1) above was produced in the same manner as in (1) above, except that 0.7 g of HPC (NISSO HPC-SL; produced by Nippon Soda Co., Ltd., viscosity: about 3 to 6 mPa·s in 2% aqueous solution at 20°C) was added in place of 1.7 g of the HPMC, and the amount of the PVA copolymer was changed from 32.3 g to 33.3 g. The hard capsule produced in this manner was named "Example 6."

A hard capsule of the same size as that obtained in (1) above was produced in the same manner as in (1) above, except that 1.7 g of HPC was used in place of 1.7 g of the HPMC. The hard capsule produced in this manner was named "Example 7."

A hard capsule of the same size as that obtained in (1) above was produced in the same manner as in (1) above, except that 3.4 g of HPC was used in place of 1.7 g of the HPMC, and the amount of the PVA copolymer was changed from 32.3 g to 30.6 g. The hard capsule produced in this manner was named "Example 8."

A hard capsule of the same size as that obtained in (1) above was produced in the same manner as in (1) above, except that 5.1 g of HPC was used in place of 1.7 g of the HPMC, and the amount of the PVA copolymer was changed from 32.3 g to 28.9 g. The hard capsule produced in this manner was named "Example 9."

A hard capsule of the same size as that obtained in (1) above was produced in the same manner as in (1) above, except that 6.8 g of HPC was used in place of 1.7 g of the HPMC, and the amount of the PVA copolymer was changed from 32.3 g to 27.2 g. The hard capsule produced in this manner was named "Example 10."

A hard capsule of the same size as that obtained in (1) above was produced in the same manner as in (1) above, except that 1.7 g of MC (METOLOSE (registered trademark) SM-4 produced by Shin-Etsu Chemical Co., Ltd.; viscosity: about 4 mPa·s in a 2% aqueous solution at 20°C) was used in place of 1.7 g of the HPMC. The hard capsule produced in this manner was named "Example 11."

A hard capsule of the same size as that obtained in (1) above was produced in the same manner as in (1) above, except that 1.7 g of dextrin (Dextrin, produced by Nacalai Tesque, Inc.) was used in place of 1.7 g of the HPMC. The hard capsule produced in this manner was named "Example 12."

A hard capsule of the same size as that obtained in (1) above was produced in the same manner as in (1) above, except that 1.7 g of polyvinylpyrrolidone (PLASDONE (registered trademark) S-630, produced by ISP TECHNOLOGIES, INC.; molecular weight measured by a light-scattering analytical method: about 58,000) was used in place of 1.7 g of the HPMC. The hard capsule produced in this manner was named "Example 13."

A hard capsule of the same size as that obtained in (1) above was produced in the same manner as in (1) above, except that 1.7 g of polyvinylpyrrolidone (PLASDONE (registered trademark) K-29/32, produced by ISP TECHNOLOGIES, INC.; molecular weight measured by a light-scattering analytical method: about 58,000) was used in place of 1.7 g of the HPMC. The hard capsule produced in this manner was named "Example 14."

A hard capsule of the same size as that obtained in (1) above was produced in the same manner as in (1) above, except that 3.4 g of polyvinylpyrrolidone (K-29/32) was used in place of 1.7 g of the HPMC, and the amount of the PVA copolymer was changed from 32.3 g to 30.6 g. The hard capsule produced in this manner was named "Example 15."

(4) For comparison, a hard capsule of the same size as that obtained in (1) above was produced in the same manner as in (1) above, except that 1.7 g of the HPMC was not used, and the amount of the PVA copolymer was changed from 32.3 g to 34.0 g. The hard capsule produced in this manner was named "Comparative Example 1."

A hard capsule of the same size as that obtained in (1) above was produced in the same manner as in (1) above, except that 1.7 g of the HPMC was not used. The hard capsule produced in this manner was named "Comparative Example 2."

A hard capsule of the same size as that obtained in (2) above was produced in the same manner as in (2) above, except that 3.4 g of the HPMC was not added. The hard capsule produced in this manner was named "Comparative Example 3."

2. Evaluation test

(1) Viscosity of starting solutions for preparing a capsule

The starting solutions for preparing a capsule having a temperature of 55°C produced by the methods described above were measured for viscosity using a B-type viscometer (produced by FUNGILAB S.A., a digital rotational viscometer, VISCO STAR-L). A No. 3 rotor was first attached to initiate the measurement. When the resistance was either overly low or overly high, the number of rotations or the rotor number was sequentially changed to carry out the measurement. The measuring time was adjusted to about 20 to 60 seconds, during which time the viscosity reaches a certain value. Table 1 shows the results.
Table 1

| Capsule | Compound Name | Addition Concentration | Solids Concentration | Viscosity (mPa·s) |
|---|---|---|---|---|
| Comp. Ex. 1 | - | - | 17% | 1540 |
| Comp. Ex. 2 | - | - | 16.3% | 1500 |
| Comp. Ex. 3 | - | - | 15.6% | 1450 |
| Example 1 | HPMC | 5% | 17% | 1300 |
| Example 2 | HPMC | 10% | 17% | 1170 |
| Example 3 | HPMC | 15% | 17% | 900 |
| Example 4 | HPMC | 5% | 20% | 2500 |
| Example 5 | HPMC | 40% | 17% | 300 |
| Example 6 | HPC | 2% | 17% | 1550 |
| Example 7 | HPC | 5% | 17% | 1420 |
| Example 8 | HPC | 10% | 17% | 910 |
| Example 9 | HPC | 15% | 17% | 870 |
| Example 10 | HPC | 20% | 17% | 660 |
| Example 11 | MC | 5% | 17% | 1350 |
| Example 12 | Dextrin | 5% | 17% | 1420 |
| Example 13 | PVP S-630 | 5% | 17% | 1180 |
| Example 14 | PVP K-29/32 | 5% | 17% | 1360 |
| Example 15 | PVP K-29/32 | 10% | 17% | 1230 |

Table 1 confirms that the starting solutions for preparing a capsule produced in the Examples had lower viscosities than those produced in the Comparative Examples.
When a water-soluble polymer was not added, the starting solution for preparing a capsule had a viscosity of about 1,500 mPa·s. However, it was confirmed that the addition of a water-soluble polymer reduced the viscosity. It was also confirmed that by increasing the amount of the water-soluble polymer, the viscosity was reduced to 1,000 mPa·s or less.
In Example 4, although the solids concentration was increased to 20%, the viscosity was sufficient to prepare a capsule, i.e., 2,500 mPa·s. This confirms that the addition of a water-soluble polymer (HPMC) to a starting solution for preparing a capsule can reduce the viscosity of the starting solution, and can increase the solids concentration of the starting solution without impairing the operability. Thereby, a thick capsule can be produced even when a starting solution that is made to adhere to a molding pin is used in a small amount, allowing the production of a capsule to be performed more easily. Further, as described below, in Example 4, where the solids concentration was increased to 20%, the drying rate was very fast, which is advantageous.

(2) Drying efficiency during capsule preparation

A stainless steel pin was immersed in and withdrawn from each of the starting solutions for preparing a capsule, and each molding pin to which the solution adheres was placed in a dryer at 60°C. After being dried for 30 minutes, 40 minutes, or 50 minutes, the film was quickly removed from each of the molding pins, and each weight was accurately measured.

Subsequently, each of the sample capsules was placed in a dryer at 105°C, and dried for 2 hours. Then, the resulting samples were allowed to cool in a desiccator (containing a silica gel), and the weight of each sample was measured again.

From a weight difference between the weight measured before drying at 105°C (hereinafter, referred to as the "weight before drying") and the weight measured after drying at 105°C (hereinafter, referred to as the "weight after drying"), the loss on drying (%) was calculated using the following formula:

$$\text{Loss on drying (\%)} = [(\text{weight before drying} - \text{weight after drying})/\text{weight before drying}] \times 100$$

A smaller value of the loss on drying indicates a faster drying rate. With respect to each of the capsule films, the measurement was performed 3 times under respective conditions, and the average value was calculated.

Table 2 shows the results.

Table 2

| Capsule | Compound Name | Addition Concentration | Solids Concentration | Loss on Drying (%) | | |
|---|---|---|---|---|---|---|
| | | | | Drying Time: 30 mins | Drying Time: 40 mins | Drying Time: 50 mins |
| Comp. Ex. 1 | - | - | 17% | 16 | 14 | 10 |
| Comp. Ex. 2 | - | - | 16.3% | 13 | 11 | 9 |
| Comp. Ex. 3 | - | - | 15.6% | 12 | 10 | 8 |
| Example 1 | HPMC | 5% | 17% | 14 | 10 | 9 |
| Example 2 | HPMC | 10% | 17% | 16 | 12 | 9 |
| Example 3 | HPMC | 15% | 17% | 10 | 10 | 7 |
| Example 4 | HPMC | 5% | 20% | 7 | 4 | 4 |
| Example 5 | HPMC | 40% | 17% | 12 | 6 | 7 |
| Example 6 | HPC | 2% | 17% | 11 | 9 | 7 |
| Example 7 | HPC | 5% | 17% | 9 | 8 | 7 |
| Example 11 | MC | 5% | 17% | 10 | 9 | 5 |
| Example 12 | Dextrin | 5% | 17% | 11 | 9 | 8 |
| Example 13 | PVP S-630 | 5% | 17% | 14 | 12 | 8 |
| Example 14 | PVP K-29/32 | 5% | 17% | 10 | 7 | 7 |
| Example 15 | PVP K-29/32 | 10% | 17% | 9 | 7 | 7 |

Table 2 confirms that those obtained in the Examples achieved faster drying rates than those obtained in the Comparative Examples. In particular, the results of Example 4 showed remarkable effects.

(3) Stability of hard capsule under high humidity

A panelist grasped, with fingers, three hard capsules each obtained above (drying time: 50 minutes), which had been stored for three days under the conditions of a temperature of 25°C and 75% RH (relative humidity); and the state of each capsule was observed and evaluated.

i: No weakness in strength is observed

ii: Usable hardness

iii: Slightly weakened in strength, causing no practical problems

vi: Very weakened in strength

Table 3

| Capsule | Compound Name | Addition Concentration | Solids Concentration | State of Hard Capsule |
|---|---|---|---|---|
| Comp. Ex. 1 | - | - | 17% | vi |
| Example 1 | HPMC | 5% | 17% | iii |
| Example 2 | HPMC | 10% | 17% | ii |
| Example 3 | HPMC | 15% | 17% | ii |
| Example 4 | HPMC | 5% | 20% | iii |
| Example 5 | HPMC | 40% | 17% | i |

Table 3 confirms that with respect to the capsules obtained with the addition of HPMC, the weakening in strength caused by high humidity can be inhibited.

(4) Solubility test for hard capsule

The hard capsules of Examples 1 and 4 obtained in 1 above were evaluated for solubility in accordance with the process of the purity test described in the Item "Capsules" of the Japanese Pharmacopoeia Fifteenth Edition. Specifically, each hard capsule was separated into a cap and a body, and one hard capsule (one pair of a cap and a body) each was placed into a 50-mL amount of water having a temperature of $37\pm2°C$, which was sometimes stirred. Then, the time taken for complete dissolution was measured. When the time was 10 minutes or less, the solubility was considered to be satisfactory.
Table 4 shows the results. In Table 4, the "Carrageenan Concentration" is on a dry weight basis, and refers to the weight of carrageenan, relative to the total amount of the film; and the "Potassium Chloride Concentration" is on a dry weight basis, and refers to the weight of potassium chloride, relative to the total amount of the film.
Table 4

| Capsule | Compound Name | Addition Concentration | Solids Concentration | Carrageenan Concentration | Potassium Chloride Concentration | Dissolution Time(min) |
|---|---|---|---|---|---|---|
| Comp. Ex. 1 | - | - | 17% | 1% | 1% | 6.5 |
| Example 1 | HPMC | 5% | 17% | 1% | 1% | 5.5 |
| Example 4 | HPMC | 5% | 20% | 0.6% | 0.6% | 5.0 |

Table 4 confirms that the dissolution time of the hard capsules of the present invention was shorter than that of the hard capsule of Comparative Example 1. The hard capsule of Example 4, where a reduced amount of a gelling agent was used, achieved an even shorter dissolution time.

(5) Impact strength test for hard capsule

Ten hard capsules each were measured for impact strength after storage in a thermohygrostat for 3 days at 25°C, 50% RH (relative humidity), using an impact strength testing machine (a capsule hardness tester; Qualicaps Co., Ltd.) shown in Fig. 1. Specifically, a 50-g weight was vertically dropped from 5 cm above an empty capsule, and the number of damaged capsules was counted. The weight was in the form of a rectangular parallelepiped (height: 4 cm; width: 1.5 cm; and depth: 3 cm). When the cracking of a capsule was confirmed with the naked eye, the capsule was considered to be damaged (broken). Table 5 shows the results.

Table 5

| Capsule | Compound Name | Addition Concentration | Solids Concentration | Loss on Drying (%) | Impact Strength (the number of broken capsules/the number of sample capsules) |
|---|---|---|---|---|---|
| Comp. Ex. 1 | - | - | 17% | 5.59 | 1/10 |
| Example 1 | HPMC | 5% | 17% | 5.58 | 0/10 |
| Example 2 | HPMC | 10% | 17% | 5.44 | 0/10 |
| Example 3 | HPMC | 15% | 17% | 5.43 | 1/10 |
| Example 4 | HPMC | 5% | 20% | 5.57 | 0/10 |

As shown in Table 5, the addition of a water-soluble polymer did not reduce the mechanical strength of the hard capsule that was made mainly of PVA copolymer.

(6) Stability test for capsule filled with solvent

0.2 mL each of polyethylene glycol having a weight average molecular weight of 400 (hereinafter referred to as "PEG 400") was used to fill two capsules each, and each of the capsules was band-sealed using a PVA copolymer aqueous solution (concentration: 21.5 mass%). Then, the resulting capsules were stored for 6 months at 40°C while stoppered, and changes in appearance, such as a change in the capsule shape, and leakage, as well as the presence of cracking, were observed with the naked eye, so as to examine the stability of the capsules when filled with a solvent. Note that polyethylene glycol is a solvent that can dissolve a poorly soluble drug. When polyethylene glycol is filled in a known hard capsule (e.g., a gelatin capsule), the moisture in the film would migrate into the polyethylene glycol, causing the capsule to break. Table 6 shows the results.

The appearance was evaluated in accordance with the following criteria:

a: No change in appearance
b: Slight appearance change, causing no practical problems
c: Significant appearance change; thus, practically unusable

Table 6

| Capsule | Compound Name | Addition Concentration | Solids Concentration | Appearance | Leakage (the number of capsules with leakage/the number of sample capsules) |
|---|---|---|---|---|---|
| Comp. Ex. 1 | - | - | 17% | a | 0/2 |
| Example 1 | HPMC | 5% | 17% | a | 0/2 |
| Example 2 | HPMC | 10% | 17% | a | 0/2 |
| Example 3 | HPMC | 15% | 17% | a | 0/2 |

(continued)

| Capsule | Compound Name | Addition Concentration | Solids Concentration | Appearance | Leakage (the number of capsules with leakage/the number of sample capsules) |
|---|---|---|---|---|---|
| Example 5 | HPMC | 40% | 17% | b | 0/2 |
| Example 6 | HPC | 2% | 17% | a | 0/2 |

Table 6 confirms that the hard capsules of the present invention exhibited sufficient stability even when filled with PEG 400.

3. Production and evaluation of hard capsule comprising triacetin

111 g of PVA (Type: EG-25; average polymerization degree: 1,700; saponification degree: 88%; produced by Nippon Synthetic Chemical Industry Co., Ltd.) and 662 g of ion exchange water were introduced into a separable flask equipped with a cooling reflux tube, a dropping funnel, a thermometer, a nitrogen inlet tube, and a stirrer. The mixture was dispersed at an ordinary temperature, and then completely dissolved at 95°C. Subsequently, 3.5 g of acrylic acid and 24.2 g of methyl methacrylate were added thereto, and after the flask was purged with nitrogen, the temperature was increased to 50°C. Then, 8.5 g of tertiary butyl hydroperoxide and 8.5 g of sodium erythorbate were added thereto, and the reaction was terminated after 4 hours, thereby obtaining a PVA copolymer aqueous solution.

Further, 5.1 g of HPMC (TC-5 (registered trademark) R produced by Shin-Etsu Chemical Co., Ltd.) was dissolved in 41 g of purified water; and 152 g of the PVA copolymer aqueous solution obtained above, 1.4 g of triacetin, 0.34 g of kappa carrageenan, and 0.34 g of potassium chloride were added thereto to yield a starting solution for preparing a capsule. The yielded solution was kept warm at about 60°C, and a stainless steel pin at room temperature was immersed and withdrawn to thereby produce a size No. 3 hard capsule having a film thickness of about 0.06 to 0.15 mm. The starting solution for preparing a capsule had a Solids Concentration of 17.4%; and with respect to the produced hard capsule, the Addition Concentration of the HPMC was 15%, and the Addition Concentration of triacetin was 4%.

The thus-produced hard capsules were evaluated for stability under high humidity, solubility, impact strength, and stability when a capsule is filled with a solvent. Specifically, each evaluation was performed in a manner similar to the methods described in (3), (4), (5), and (6) in "2. Evaluation test." The results are shown below:

Stability under high humidity: the state of the hard capsule was "iii"
Solubility: 9.5 minutes (which is within 10 minutes or less; thus, the solubility is satisfactory)
Impact strength (the number of broken capsules/the number of sample capsules): 0/10
Stability when filled with a solvent: Appearance "a"
Leakage (the number of capsules with leakage/the number of sample capsules) "0/2"

The above results confirm that the stability, solubility, and impact resistant were also maintained with respect to the hard capsule comprising triacetin.

**Claims**

1. A hard capsule having a film comprising:

(A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof; and
(B) at least one water-soluble polymer selected from the group consisting of cellulose polymers, dextrin, and polyvinylpyrrolidone.

**2.** The hard capsule according to claim 1,
wherein the water-soluble polymer of (B) is at least one member selected from the group consisting of hydroxypropyl methyl cellulose, hydroxypropyl cellulose, methyl cellulose, hydroxyethyl cellulose, dextrin, and polyvinylpyrrolidone.

**3.** The hard capsule according to claim 1 or 2, comprising 2 to 100 parts by weight of the water-soluble polymer of (B), relative to 100 parts by weight of the polymer or copolymer of (A).

**4.** The hard capsule according to any of claims 1 to 3, wherein the film further comprises (C) a gelling agent.

**5.** The hard capsule according to any of claims 1 to 4, which is to be filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,
(f) aliphatic alcohols having 6 to 12 carbon atoms, and
(g) polyoxyethylene sorbitol fatty acid esters.

**6.** A hard capsule formulation comprising the hard capsule of any of claims 1 to 5, wherein the hard capsule is filled with at least one member selected from the group consisting of

(a) polyethylene glycols having a weight average molecular weight of 2,000 or less, or derivatives thereof,
(b) polyoxyethylene sorbitan fatty acid esters,
(c) fatty acids having 6 to 12 carbon atoms or salts thereof,
(d) polyoxyethylene castor oil,
(e) diethylene glycol ether derivatives,
(f) aliphatic alcohols having 6 to 12 carbon atoms, and
(g) polyoxyethylene sorbitol fatty acid esters.

**7.** A method for producing a film of the hard capsule of any of claims 1 to 5, comprising:

forming an aqueous solution containing (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof, and (B) at least one water-soluble polymer selected from the group consisting of cellulose polymers, dextrin, and polyvinylpyrrolidone, into a capsule form by drying.

**8.** Use of (A) a polymer or copolymer obtained by polymerizing or copolymerizing at least one polymerizable vinyl monomer represented by Formula (1):

$$H_2C = C(R_1)\text{-}COOR_2 \qquad (1)$$

wherein $R_1$ represents hydrogen or methyl, and $R_2$ represents hydrogen or alkyl having 1 to 4 carbon atoms, in the presence of polyvinyl alcohol and/or a derivative thereof; and (B) at least one water-soluble polymer selected from the group consisting of cellulose polymers, dextrin, and polyvinylpyrrolidone, in producing a hard capsule.

Fig. 1

Weight 50g

5 cm

Empty Capsule

## INTERNATIONAL SEARCH REPORT

| | International application No. |
|---|---|
| | PCT/JP2010/056102 |

A.  CLASSIFICATION OF SUBJECT MATTER
*A61K47/32*(2006.01)i, *A61K9/48*(2006.01)i, *A61K47/02*(2006.01)i, *A61K47/10*
(2006.01)i, *A61K47/12*(2006.01)i, *A61K47/34*(2006.01)i, *A61K47/36*(2006.01)i,
*A61K47/38*(2006.01)i
According to International Patent Classification (IPC) or to both national classification and IPC

B.  FIELDS SEARCHED

Minimum documentation searched (classification system followed by classification symbols)
A61K47/32, A61K9/48, A61K47/02, A61K47/10, A61K47/12, A61K47/34,
A61K47/36, A61K47/38

Documentation searched other than minimum documentation to the extent that such documents are included in the fields searched
| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>A | WO 2006/70578 A1 (Qualicaps Co., Ltd.),<br>06 July 2006 (06.07.2006),<br>claims 1 to 14; paragraphs [0021], [0022],<br>[0051] to [0053]; example 4<br>& US 2008/0008750 A1 & EP 1832282 A1 | 1-3,5,6,8<br>4,7 |
| X<br>A | JP 2007-230948 A (Meijo University, Nisshin<br>Kasei Co., Ltd.),<br>13 September 2007 (13.09.2007),<br>claims 1 to 3, 5<br>(Family: none) | 8<br>1-7 |

☒  Further documents are listed in the continuation of Box C.　　☐  See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>11 May, 2010 (11.05.10) | Date of mailing of the international search report<br>18 May, 2010 (18.05.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/056102

C (Continuation). DOCUMENTS CONSIDERED TO BE RELEVANT

| Category* | Citation of document, with indication, where appropriate, of the relevant passages | Relevant to claim No. |
|---|---|---|
| Y | JP 61-100519 A (Shin-Etsu Chemical Co., Ltd.), 19 May 1986 (19.05.1986), claim 1; page 3, upper left column, lines 3 to 9, upper right column, line 4 to lower left column, line 1 & US 4917885 A     & EP 180287 A2 | 1-8 |
| Y | JP 2001-170137 A (Shionogi Qualicaps Co., Ltd.), 26 June 2001 (26.06.2001), claims 1 to 7; paragraphs [0002] to [0009] (Family: none) | 1-8 |
| Y | WO 2002/17848 A1 (Nisshin Kasei Co., Ltd.), 07 March 2002 (07.03.2002), claims 1 to 14; page 1, the last line to page 2, line 9; page 5, line 27 to page 6, line 7 & US 2003/0166763 A1   & EP 1323404 A1 & CN 1449272 A | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

* WO 2002017848 A

**Non-patent literature cited in the description**

* *Pharmaceutical Technology Europe,* October 1998, vol. 84 (86), 88-90